(19) Europäisches Patentamt
European Patent Office
Office européen des brevets

(11) Publication number: 0 102 343 B1

# (12) EUROPEAN PATENT SPECIFICATION

(45) Date of publication of patent specification: 26.02.86

(51) Int. Cl.[4]: C 07 C 121/43, C 07 C 120/00

(21) Application number: 83870084.7

(22) Date of filing: 25.08.83

## (54) Process for producing nitrilotriacetonitrile.

(30) Priority: 30.08.82 US 413057

(43) Date of publication of application:
07.03.84 Bulletin 84/10

(45) Publication of the grant of the patent:
26.02.86 Bulletin 86/09

(84) Designated Contracting States:
BE DE FR GB IT NL

(56) References cited:
EP-A-0 102 935
GB-A-1 334 991
US-A-3 061 628
US-A-3 907 858

(73) Proprietor: MONSANTO COMPANY
Patent Department 800 North Lindbergh Boulevard
St. Louis, Missouri 63166 (US)

(72) Inventor: Shen, Chung Yu
12630 Conway Downs Drive
St. Louis Missouri 63141 (US)

(74) Representative: Lunt, John Cooper et al
Monsanto Europe S.A. Patent Department
Avenue de Tervuren 270-272 Letter Box No 1
B-1150 Brussels (BE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

**Description**

Background of the invention

Field of the invention

This invention relates to the production of nitrilotriacetonitrile, and more particularly to the source and manner of addition of hydrogen cyanide to the reaction mixture to produce nitrilotriacetonitrile.

Background

It is well known that ammonia, an ammonium salt, or an ammonia-formaldehyde condensation product will react with formaldehyde and hydrogen cyanide to produce nitrilotriacetonitrile. A common ammonia-formaldehyde condensation product is hexamethylenetetramine (HMTA). These reactions have been generally defined in the following U.S. Patents to J. J. Singer, et al: 2,855,428, and 3,061,628 and to Morgan, et al: 3,463,805.

Nitrilotriacetonitrile can be produced from hexamethylenetetramine by the following reaction:

$$N_4(CH_2)_6+6CH_2O+12HCN \xrightarrow{pH\leq 1} 4N(CH_2CN)_3+6H_2O \quad (1)$$

If the hexamethylenetetramine is replaced by equivalent amounts of ammonia or ammonium and formaldehyde, nitrilotriacetonitrile can be produced from ammonia or an ammonium salt by the following reaction:

$$NH_3(\text{or } HN_4^+)+3CH_2O+3HCN \xrightarrow{pH\leq 1} N(CH_2CN)_3(+H^+ \quad (2)$$

if $NH_4^+$ is used

The effect of many factors on these reactions has been studied, including dilution ratios, temperature ranges, order of addition of reactants, order of reaction steps, and others. But, current processes use hydrogen cyanide as commercially available, which is anhydrous or substantially anhydrous form.

Hydrogen cyanide is most commonly produced commercially by reaction of ammonia with methane, methanol or other suitable volatile hydrocarbon, at elevated temperatures to produce hydrogen cyanide, hydrogen, and other inert gases.

Hydrogen cyanide is normally purified to greater than 98% purity and liquified prior to use.

This purification process typically comprises cooling the product gas stream; removing ammonia with a sulfuric acid solution or a monoammonium phosphate solution; absorbing hydrogen cyanide from the resulting gas stream in an acidic aqueous solution; stripping the hydrogen cyanide from the resulting solution; condensing the stripped hydrogen cyanide and water mixture; fractionating to produce hydrogen cyanide; liquifying the hydrogen cyanide, and stabilizing the liquified hydrogen cyanide to prevent polymerization.

Purification of hydrogen cyanide to the commercial grade anhydrous hydrogen cyanide as outlined above, is a complex process requiring large expenditures for equipment, energy, and material handling. Additionally, cyanide-containing waste streams are produced that must be treated and disposed of. It would be an advancement in the art to be able to use hydrogen cyanide to produce nitrilotriacetonitrile without the costly and waste producing purification steps.

It is an advantage of this invention that nitrilotriacetonitrile can be produced using hydrogen cyanide without undergoing the usual purification.

It is a further advantage of this invention that mother liquor produced in production of nitrilotriacetonitrile can be recycled to extract hydrogen cyanide from the reactor product gas stream reducing the amount of waste in the nitrilotriacetonitrile and hydrogen cyanide production processes.

Further advantages can be seen from this following description and examples.

Summary of the invention

This invention provides a process for producing nitrilotriacetonitrile comprising:

a. producing a gas stream containing hydrogen cyanide;

b. scrubbing said gas stream with an aqueous solution containing nitrilotriacetonitrile mother liquor at a temperature above 7°C, to produce a hydrogen cyanide-containing solution;

c. adding formaldehyde and an ammonia derivative selected from the group consisting of ammonia, an ammonium salt and hexamethylenetetramine, to said hydrogen cyanide-containing solution, to produce a reaction mixture; and

d. reacting said reaction mixture to produce nitrilotriacetonitrile.

Description of the preferred embodiment

Hydrogen cyanide can be produced by the reaction of methane, methanol, or other volatile hydrocarbon with ammonia in the presence of a catalyst or in a fluidized bed at a temperature in excess of 1000°C. The gas stream produced contains nitrogen, methane, ammonia, hydrogen, hydrogen cyanide, and other gas components. Unless otherwise specified, all parts and percents are by weight.

Hydrogen cyanide is produced commercially primarily by the Andrussow Process, the Degussa Process, also called the BMA Process, and the Fluohmic Process, and ammoxidation of methanol or other hydrocarbons. All of these processes use ammonia as the source of nitrogen, and the product gas stream will contain some unreacted ammonia. Since ammonia will react with hydrogen cyanide, removal of this unreacted ammonia is an important step in the purification process.

The Andrussow Process produces hydrogen cyanide by the following theoretical reaction:

$$CH_4+NH_3+1.5O_2 \longrightarrow HCN+3H_2O \quad (3)$$

at about 1100°C, with heat being supplied by combustion of methane. Metal catalysts from Group VIII of the Periodic Table are normally used, preferably a Platinum-Rhodium gauze. The oxygen is normally supplied by mixing an appropriate amount of air with the other reactants. A typical product gas stream produced by the Andrussow Process has the following composition: nitrogen 64%, water 13%, hydrogen cyanide 11%, carbon monoxide 7%, hydrogen 2%, ammonia 2%, carbon dioxide 1%, methane 0.4%.

The Degussa Process produces hydrogen cyanide by the following theoretical reaction:

$$CH_4+NH_3 \longrightarrow HCN+3H_2 \quad (4)$$

in a tubular reactor impregnated with a platinum catalyst at 1200°C, with heat being supplied by indirectly heating the tubes with gas or electricity. A typical product gas stream contains: hydrogen cyanide 71%, hydrogen 17%, nitrogen 7%, methane 4%, ammonia 2%.

Other processes for production of hydrogen cyanide include the Fluohmic Process; by reacting ammonia and propane in an electrically heated fluidized carbon bed; ammoxidation of methanol or other hydrocarbons; and a decomposition of formamide, which is produced from ammonia and carbon monoxide. Any process that produces hydrogen cyanide in sufficient quantities can be used with this invention.

Under the process of this invention, an unpurified hydrogen cyanide-containing gas stream, or a hydrogen cyanide-containing gas stream from which ammonia has been scrubbed, is scrubbed with nitrilotriacetonitrile mother liquor. Preferred is mother liquor containing sulfuric acid, and preferably about 10% to about 25% apparent sulfuric acid concentration. Apparent sulfuric acid concentration is determined by titration against a standard sodium hydroxide solution. Additional sulfuric acid and additional water can be added to the mother liquor to form the scrubbing solution and to adjust the apparent sulfuric acid concentration to the desired concentration. The unpurified gas stream may be cooled prior to scrubbing, preferably to about 80°C to about 100°C, or may be both cooled and treated to remove the unreacted ammonia. The resulting solution preferably contains about 10% to about 60%, more preferably about 20% to about 40%, hydrogen cyanide. This hydrogen cyanide-containing solution can be used without further purification to produce nitrilotriacetonitrile, using any suitable process for synthesizing nitrilotriacetonitrile.

Production of nitrilotriacetonitrile involves crystallization of the product from the reactant solution resulting in production of mother liquor containing an inorganic acid. This mother liquor can be used for scrubbing the hydrogen cyanide from the reactor stream. Additional acid and additional water can be added as necessary to make up a suitable scrubbing solution.

Example 1

A typical sample of nitrilotriacetonitrile mother liquor was analyzed to contain about 10% apparent sulfuric acid, about 1% ammonia, and about 10% organic components comprising unreacted organic reactants, nitrile intermediates, and dissolved nitrilotriacetonitrile. A mixture of the mother liquor and hydrogen cyanide at a ratio of 1 to 2 was homogeneous. Upon cooling to about 7°C, the mixture formed two layers, an upper hydrogen cyanide layer and a lower mother liquor layer. Upon warming above about 7°C, a homogeneous solution again formed. Because the solubility of the hydrogen cyanide in the mother liquor below about 7°C decreases significantly, hydrogen cyanide scrubbing with nitrilotriacetonitrile mother liquor must be done at temperatures above about 7°C.

Example 2

The scrubbing efficiency of hydrogen cyanide by a dilute sulfuric acid solution and by a dilute sulfuric acid solution containing nitrilotriacetonitrile mother liquor were determined at about 44°C and at about 32°C. Five percent hydrogen cyanide in an inert gas was bubbled at a rate of about 10 milliliters per minute into 100 milliliters of solution, through 5.5 centimeters of the solution, in a constant temperature water bath, until the scrubbed gas contained about 1% hydrogen cyanide. By these tests, the dilute sulfuric acid solution and the nitrilotriacetonitrile mother liquor showed similar scrubbing efficiencies, even though theoretically the hydrogen cyanide should be somewhat more soluble in the dilute sulfuric acid solution than in the mother liquor. At about 44°C, the hydrogen cyanide concentration of the scrubbing solution reached about 25%, and at about 32°C, the hydrogen cyanide concentration of the solution reached about 57%.

Example 3

An apparatus was constructed for continuous production of nitrilotriacetonitrile, as described below. The components were constructed using stainless steel.

A dual feed system was constructed consisting of two feed tanks each with a feed pump. The feed from the two pumps joined at a tee. A small gear pump and mixing loop premixed the reactants to form the reaction mixture just prior to its introduction to the reactor system.

The first stage of the reactor system was a circulating loop reactor. The circulating loop reactor consisted of a pump, two shell and tube heat exchangers, and connecting lines to form a continuous loop, an inlet for introduction of the reaction mixture to the loop and an outlet through which the reaction mixture can be withdrawn. One heat exchanger was 5 inches (12.7 cm) in length, and the other was 7 inches (17.8 cm) in length. Both were constructed of 1/4" (6.35 mm) OD tubing that was jacketed for heat transfer fluid. Flow of the heat transfer fluid was thermostatically controlled to maintain the desired temperature. The volume of the circulating loop reactor was 10 ml.

A plug-flow tubular reactor was connected to the outlet from the circulating loop reactor as the second stage of the reactor system. The plug-flow tubular reactor consisted of two sections, a packed section and a heated return section. The packed section was a shell and tube heat exchanger made using 2 feet (61.0 cm) of 1/2" (12.7 mm) OD tubing packed with glass beads and jacketed for heat transfer fluid. The volume of the packed section was 30 ml. The heated return connected the end of the plug-flow tubular reactor to the product receiver. The heated return was another shell and tube heat exchanger constructed of 1/8" (3.2 mm) OD tubing with a jacket. The volume of the heated return was 5 ml, for a total reactor system volume of 45 ml. The packed section and the heated return section were both supplied with a heat transfer fluid, the flow of which was thermostatically controlled to maintain the temperature. The connection between the packed section, the heated return, and the product receiver were wrapped with heating tape to insure that the temperature remained above 95°C to prevent pluggage caused by crystallized nitrilotriacetonitrile.

The product receiver was stirred, was cooled by a bucket of ice and water to crystallize the nitrilotriacetonitrile, and was fitted with a valve at the bottom to remove the nitrilotriacetonitrile slurry produced. Crystalline nitrilotriacetonitrile was separated by filtration.

The reactor system and the receiver were all maintained at a pressure of about 75 psig (about 620 KPa) with pressurized nitrogen.

The two feed tanks contained reactant solutions consisting of:

| Feed No. 1 | Feed No. 2 |
|---|---|
| 56 g 99.7% HMTA | 23 g 96.4% $H_2SO_4$ |
| 211 g 35.9% $CH_2O$ | 131 g mother liquor (containing 9.23% $H_2SO_4$) |
| 9 g $H_2O$ | 36 g $H_2O$ |
| | 138 g 99% HCN |

Feed No. 2 contained 42% hydrogen cyanide, equivalent to a solution produced by scrubbing a hydrogen cyanide-containing gas stream at about 35°C. Feed No. 2 contained about 10.4% apparent sulfuric acid. The feed rates were adjusted to produce the amount of excess hydrogen cyanide indicated. Formaldehyde was present in about 5% excess as indicated. Sojourn times were calculated by dividing the reactor volume by the total feed rate. Since the volume of the circulating loop reactor was approximately 22% of the volume of the complete reactor system, the reaction mixture on the average spent about 22% of the sojourn time in the circulating loop reactor. The reaction conditions and the yield of nitrilotriacetonitrile (NTAN) for each of the reaction runs is shown in Table I:

TABLE I

| | Run No. | | | | | |
|---|---|---|---|---|---|---|
| | 1 | 2 | 3 | 4 | 5 | 6 |
| % Excess $CH_2O$ | 5 | 5 | 5 | 5.2 | 5 | 5. |
| % Excess HCN | 11 | 0.85 | 4.7 | 10.6 | 10.4 | 16.5 |
| Reactor temp. °C | 115° | 115° | 115° | 115° | 115° | 115° |
| Sojourn time, min. | 10.2 | 10.0 | 10.0 | 10.3 | 10.4 | 10.6 |
| % Yield NTAN | 91.6 | 88.0 | 91.5 | 92.6 | 91.3 | 97.5 |
| Melting range NTAN °C | 125.5—127.2 | 125.0—126.8 | 125.5—127.3 | 125.0—126.8 | 125.3—127.9 | 125.4—127.0 |

Example 4

Nitrilotriacetonitrile was produced in three runs with the first using recycled mother liquor from stock and the second and third using recycled mother liquor from the previous run. About 50% of the mother liquor produced was recycled to the next run. The mother liquor was mixed with ammonium sulfate, sulfuric acid, and liquid hydrogen cyanide. This simulated a solution obtained by scrubbing a product gas stream containing hydrogen cyanide and ammonia with the nitrilotriacetonitrile mother liquor. Solutions of hexamethylenetetramine and formaldehyde were added to give a reaction mixture as shown in Table II. The "organics" were primarily intermediates from the nitrilotriacetonitrile reaction and dissolved nitrilotriacetonitrile from the mother liquor. The reaction mixture was reacted at a temperature of 75—80°C for about one hour. The mixture was cooled below about 20°C to crystallize nitrilotriacetonitrile. The crystalline nitrilotriacetonitrile was separated by filtration and the crystals washed. The amount of nitrilotriacetonitrile and mother liquor produced, and analysis of the mother liquor are shown in Table II.

TABLE II

| Runs | 1 | 2 | 3 |
|---|---|---|---|
| Source of Recycled Mother Liquor | Stock | Run 1 | Run 2 |
| Reactant Mixture, g. | | | |
| Apparent $H_2SO_4$ | 22.0 | 26.6 | 22.6 |
| $NH_3$ | 4.4 | 3.8 | 3.8 |
| HCN | 68.0 | 72.5 | 69.9 |
| HMTA | 28.4 | 28.4 | 28.4 |
| $CH_2O$ | 37.0 | 37.0 | 37.0 |
| Organics | 3.9 | 10.2 | 8.3 |
| $H_2O$ | 138.3 | 158.3 | 166.3 |
| Total | 306.0 | 336.6 | 336.6 |
| Products, g. | | | |
| NTAN | 98.6 | 102.2 | 100.8 |
| Mother Liquor | 207.4 | 234.4 | 235.8 |
| NTAN Yield (based on HMTA) | 90.8% | 94.1% | 92.9% |
| Analysis of Mother Liquor, g. | | | |
| Apparent $H_2SO_4$ | 21.6 | 24.9 | 22.6 |
| $NH_3$ | 3.7 | 3.4 | 2.6 |
| Organics and Unreacted HCN and $CH_2O$ | 26.4 | 10.6 | 10.6 |

In each run some ammonia was consumed, and the amount of ammonia did not build up in the mother liquor as it was recycled. These data show that ammonium salts will not build up through a series of runs with mother liquor continuously recycled.

Example 5

This example describes the process of this invention with the Degussa Process for production of hydrogen cyanide. This example follows the scheme outlined in Figure 1. Ammonia (11) and methane (12) are fed into an indirectly heated tubular reactor. The product gas stream (13) is then cooled to 80°C to 100°C. This cooled gas stream (14) contains about 71% hydrogen cyanide, with the remainder being methane, ammonia, hydrogen, and nitrogen. This cooled gas stream is then scrubbed with a solution (15) made up of recycled mother liquor from crystallization of nitrilotriacetonitrile (18) together with added sulfuric acid (19) to adjust the apparent sulfuric acid concentration of the scrubbing solution to about 18.5%. The hydrogen cyanide-free gas stream (16) is removed, and the hydrogen cyanide-containing solution (17), with about

33% hydrogen cyanide is then used in the reaction with hexamethylenetetramine and formaldehyde or with ammonia and formaldehyde to produce nitrilotriacetonitrile.

Example 6

This example describes the process of this invention with the Andrussow Process, with ammonia scrubbing prior to the hydrogen cyanide scrubbing. This example follows the scheme outlined in Figure 2. Methane (21), ammonia (22), and air (23) are fed into a reactor containing platinum-rhodium catalyst. The product gas stream (24) is then cooled to 80°C to 100°C.

This cooled gas stream (25) is then scrubbed with a solution containing about 30% monoammonium phosphate. The ammonium-containing solution (27) could be treated to recover the ammonia, or could be used directly as diammonium phosphate. The ammonia-free gas stream (28) is then scrubbed with a solution (29) made up of recycled mother liquor from crystallization of nitrilotriacetonitrile (32) together with added sulfuric acid (33) sufficient to adjust the apparent sulfuric acid concentration of the scrubbing solution to about 18.5%. The hydrogen free cyanide-containing gas stream (30) is removed, and the hydrogen cyanide-containing solution (31), with about 33% hydrogen cyanide is then used in the reaction with hexamethylenetetramine and formaldehyde or with ammonia and formaldehyde to produce nitrilotriacetonitrile.

**Claims**

1. A process for producing nitrilotriacetonitrile comprising:

a. producing a gas stream containing hydrogen cyanide;

b. scrubbing said gas stream with an aqueous solution containing nitrilotriacetonitrile mother liquor at a temperature above 7°C, to produce a hydrogen cyanide-containing solution;

c. adding formaldehyde and an ammonia derivative selected from the group consisting of ammonia, an ammonium salt, and hexamethylenetetramine, to said hydrogen cyanide-containing solution, to produce a reaction mixture; and

d. reacting said reaction mixture to produce nitrilotriacetonitrile.

2. The process of Claim 1 wherein the ammonia derivative is hexamethylenetetramine.

3. The process of Claim 1 wherein the ammonia derivative is ammonia or an ammonium salt.

4. The process of Claim 1 wherein the gas stream containing hydrogen cyanide is produced by reacting methane, ammonia, and oxygen in the presence of a metal catalyst from Group VIII of the Periodic Table.

5. The process of Claim 1 wherein the gas stream containing hydrogen cyanide is produced by reacting methane and ammonia in an indirectly heated reactor.

6. The process of Claim 1 wherein the gas stream containing hydrogen cyanide is produced by reacting ammonia and propane in an electrically heated fluidized carbon bed.

7. The process of Claim 1 wherein the aqueous scrubbing solution contains sulfuric acid.

8. The process of Claim 7 wherein the apparent concentration of sulfuric acid is about 10% to about 25%.

9. The process of Claim 1 wherein the hydrogen cyanide-containing solution contains about 10% to about 60% hydrogen cyanide.

10. The process of Claim 1 wherein the hydrogen cyanide-containing solution contains about 20% to about 40% hydrogen cyanide.

11. The process of Claim 1, further comprising treating the gas stream containing hydrogen cyanide to remove ammonia, prior to scrubbing of (b).

**Patentansprüche**

1. Verfahren zur Herstellung von Nitrilotriacetonitril, dadurch gekennzeichnet, daß man

(a) einen Cyanwasserstoff enthaltenden Gasstrom erzeugt;

(b) den Gasstrom mit einer Nitrilotriacetonitril enthaltenden Mutterlauge mit einer Temperatur von oberhalb 7°C unter Bildung einer Cyanwasserstoff enthaltenden Lösung auswäscht;

(c) zu der Cyanwasserstoff enthaltenden Lösung Formaldehyd und ein Ammoniakderivat ausgewählt aus der Ammoniak, ein Ammoniumsalz und Hexamethylentetramin umfassenden Gruppe unter Bildung einer Reaktionsmischung zusetzt; und

(d) Reaktionsmischung unter Bildung von Nitrilotriacetonitril umsetzt.

2. Verfahren nach Anspruch 1, dadurch gekennzeichnet, daß man als Ammoniumderivat Hexamethylentetramin einsetzt.

3. Verfahren nach Anspruch 1, dadurch gekennzeichnet, daß man als Ammoniumderivat Ammoniak oder ein Ammoniumsalz einsetzt.

4. Verfahren nach Anspruch 1, dadurch gekennzeichnet, daß der Cyanwasserstoff enthaltende Gasstrom durch Umsetzen von Methan, Ammoniak und Sauerstoff in Gegenwart eines Metallkatalysators aus der Gruppe VIII des Periodensystems erzeugt wird.

5. Verfahren nach Anspruch 1, dadurch gekennzeichnet, daß der Cyanwasserstoff enthaltende Gasstrom durch Umsetzen von Methan und Ammoniak in einem indirekt geheizten Reaktor erzeugt wird.

6. Verfahren nach Anspruch 1, dadurch gekennzeichnet, daß der Cyanwasserstoff enthaltende Gasstrom durch Umsetzen von Ammoniak und Propan in einer elektrisch geheizten Kohlenstoff-Wirbelschicht erzeugt wird.

7. Verfahren nach Anspruch 1, dadurch gekennzeichnet, daß die wäßrige Waschlösung Schwefelsäure enthält.

8. Verfahren nach Anspruch 7, dadurch gekennzeichnet, daß die scheinbare Konzentration der Schwefelsäure etwa 10% bis etwa 25% beträgt.

9. Verfahren nach Anspruch 1, dadurch gekennzeichnet, daß die Cyanwasserstoff enthaltende Lösung etwa 10% bis etwa 60% Cyanwasserstoff enthält.

10. Verfahren nach Anspruch 1, dadurch gekennzeichnet, daß die Cyanwasserstoff enthaltende Lösung etwa 20% bis etwa 40% Cyanwasserstoff enthält.

11. Verfahren nach Anspruch 1, dadurch gekennzeichnet, daß man den Cyanwasserstoff enthaltenden Gasstrom vor dem Auswaschen in der Stufe (b) zur Entfernung von Ammoniak behandelt.

**Revendications**

1. Procédé de production du nitrilotriacétonitrile, consistant:

a. à produire un courant gazeux contenant de l'acide cyanhydrique.

b. à laver ce courant gazeux avec une solution aqueuse contenant de la liqueur-mère du nitrilotriacétonitrile à une température supérieure à 7°C pour produire une solution contenant de l'acide cyanhydrique;

c. à ajouter du formaldéhyde et un dérivé d'ammoniac choisi dans le groupe constitué de l'ammoniac, d'un sel d'ammonium et de l'hexaméthylènetétramine à cette solution contenant de l'acide cyanhydrique, pour produire un mélange réactionnel; et

d. à faire réagir ce mélange réactionnel pour produire du nitrilotriacétonitrile.

2. Procédé selon la revendication 1, dans lequel le dérivé de l'ammoniac est l'hexaméthylènetétramine.

3. Procédé selon la revendication 1, dans lequel le dérivé de l'ammoniac est l'ammoniac ou un sel d'ammonium.

4. Procédé selon la revendication 1, dans lequel le courant gazeux contenant de l'acide cyanhydrique est produit en faisant réagir du méthane, de l'ammoniac et de l'oxygène en présence d'un catalyseur métallique du groupe VIII de la classification périodique.

5. Procédé selon la revendication 1, dans lequel le courant gazeux contenant de l'acide cyanhydrique est produit en faisant réagir du méthane et de l'ammoniac dans un réacteur chauffé indirectement.

6. Procédé selon la revendication 1, dans lequel le courant gazeux contenant de l'acide cyanhydrique est produit en faisant réagir de l'ammoniac et du propane dans un lit de carbone fluidisé chauffé électriquement.

7. Procédé selon la revendication 1, dans lequel la solution de lavage aqueuse contient de l'acide sulfurique.

8. Procédé selon la revendication 7, dans lequel la concentration apparente de l'acide sulfurique est environ 10 à environ 25%.

9. Procédé selon la revendication 1, dans lequel la solution contenant de l'acide cyanhydrique contient environ 10 à environ 60% de l'acide cyanhydrique.

10. Procédé selon la revendication 1, dans lequel la solution contenant de l'acide cyanhydrique contient environ 20% à environ 40% d'acide cyanhydrique.

11. Procédé selon la revendication 1, consistant en outre à traiter le courant gazeux contenant l'acide cyanhydrique pour éliminer l'ammoniac avant le lavage de (b).

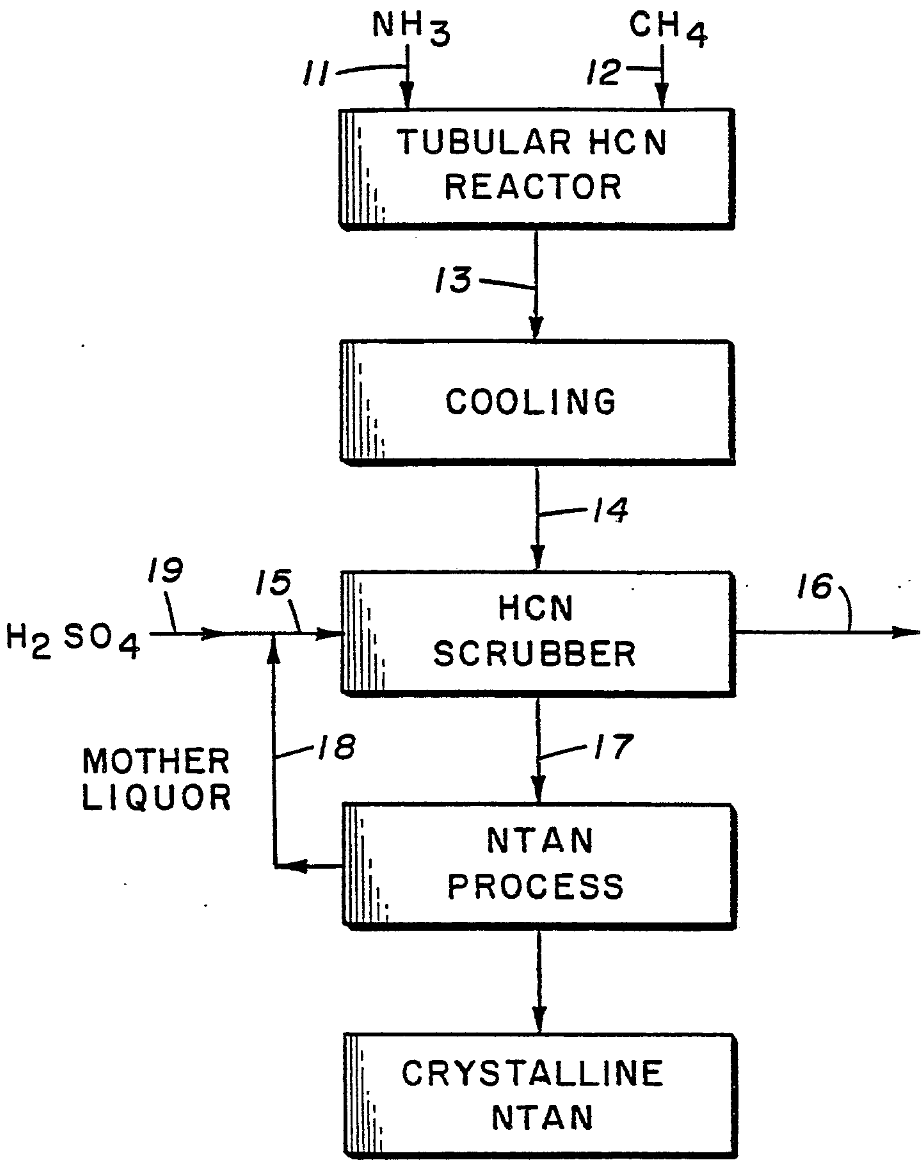
NH3
CH4
11
12
TUBULAR HCN REACTOR
13
COOLING
14
H2 SO4
19
15
HCN SCRUBBER
16
MOTHER LIQUOR
18
17
NTAN PROCESS
CRYSTALLINE NTAN

FIG. I.

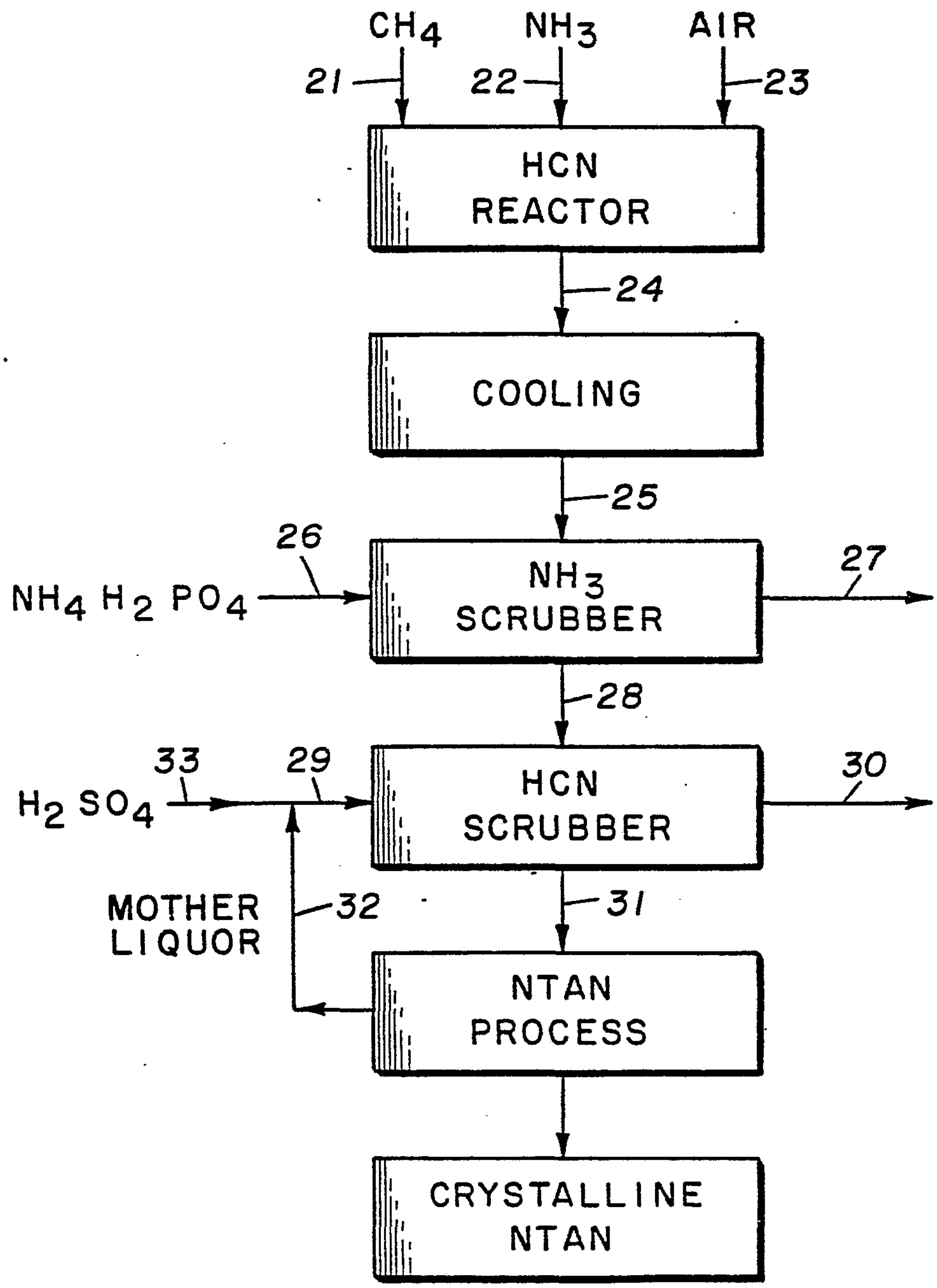

CH4
NH3
AIR
21
22
23
HCN REACTOR
24
COOLING
25
26
NH4 H2 PO4
NH3 SCRUBBER
27
28
33
29
H2 SO4
HCN SCRUBBER
30
MOTHER LIQUOR
32
31
NTAN PROCESS
CRYSTALLINE NTAN


FIG. 2.